# EUROPÄISCHE PATENTSCHRIFT

(11) **EP 0 321 606 B1**
(45) Veröffentlichungstag und Bekanntmachung des Hinweises auf die Patenterteilung: **29.09.1993**
(21) Anmeldenummer: 87119172.2
(22) Anmeldetag: 23.12.1987
(51) Int. Cl.: C07K 3/00, C12P 21/02, A61K 37/02

(54) **Zelluläre amphipatische Proteine in aggregierten Formen und Verfahren zur Herstellung und Reinigung diese Proteine**
Cellular amphipatic proteins in aggregated forms, and procedure for the production and purification of these proteins
Protéines amphipatiques cellulaires en forme d'agrégats et procédé pour la production et la purification de ces protéines

(43) Veröffentlichungstag der Anmeldung: 28.06.1989
(73) Patentinhaber: IMMUNO Aktiengesellschaft, A-1221 Wien (AT)
(72) Erfinder: Eibl, Johann, Dr., A-1180 Wien (AT); Dorner, Friedrich, Prof.Dr., A-1230 Wien (AT)
(74) Vertreter: Grünecker, Kinkeldey, Stockmair & Schwanhäusser Anwaltssozietät

(56) Entgegenhaltungen:
- EP-A- 0 233 044
- BIOTECHNOLOGY, Band 3, Nr. 6, Juni 1985, Seiten 561-566, New York, US; M.-L. MICHEL et al.: "Expression of amplified hepatitis B virus surface antigen genes in chinese hamster ovary cells"
- NATURE, Band 290, 5. März 1981, Seiten 51-54, Macmillan Journals Ltd, London, GB; J. SKELLY et al.: "Hepatitis B polypeptide vaccine preparation in micelle form"
- EUR. J. BIOCHEM., Band 155, 1986, Seiten 453-468, FEBS, Elsevier, (North Holland), NL; K. MARTINEK et al.: "Micellar enzymology"
- "METHODS IN ENZYMOLOGY", Band 104, "Enzyme Purification and Related Technique", 1984, Teil C, edited by W.B. JAKOBY, Seiten 305-318, Academic Press, Inc., Orlando, US; L.M. HJELMELAND et al.: "Solubilization of functional membrane proteins"
- "METHODS IN ENZYMOLOGY", Band 104, "Enzyme Purification and Related Technique", 1984, Teil C, edited by W.B. JAKOBY, Seiten 329-339, Academic Press, Inc., Orlando, US; J. VAN RENSWOUDE et al.: "Purification of integral membrane proteins"

## Beschreibung

Die Erfindung betrifft in Zellen produzierte, lösliche amphipatische Proteine sowie ein Verfahren zum Isolieren und Reinigen dieser Proteine.

Alle physiologisch aktiven Zellen produzieren Proteine, die sie benötigen, um ihre jeweiligen Lebensfunktionen aufrecht zu erhalten. Die synthetisierten Proteine haben verschiedene Aufgaben. Einige wirken als Enzyme, d.h. sie katalysieren die Stoffwechselreaktion in der Zelle. Andere dienen dazu, die Lebensvorgänge des Organismus, zu dem die Zelle gehört, aufeinander abzustimmen, und wieder andere bilden die Struktur dieser Zelle. Proteine, die in diesen Zellen produziert werden, können jedoch auf andere Organismen antigene Wirkungen haben, d.h. sie induzieren in diesen Organismen Immunreaktionen. Auch Viren enthalten in ihrer Hülle Proteine, die als Antigene wirken können.

Die Anweisungen für die Proteinsynthese befinden sich in den Genen der Zellen, die als funktionelle Einheiten der Erbsubstanz angesehen werden können. Es ist heute eine gut beherrschte Technik, die genetische Ausstattung einer Zelle, beispielsweise eines Bakteriums, zu verändern, indem man ein Gen, beispielsweise aus einer tierischen oder menschlichen Zelle, in eine andere Zelle, häufig eine Bakterienzelle, überträgt. Das Bakterium synthetisiert dann auch das Protein, für das das fremde, eingefügte Gen die Information enthält. Eines der bekanntesten Beispiele ist das in der Bauchspeicheldrüse des Menschen produzierte Insulin, dessen genetische Information in das Bakterium Eschericha coli übertragen wurde.

Die sowohl natürlicherweise als auch durch gentechnische Manipulation von Zellen produzierten Proteine werden entweder in den Überstand ausgeschleust oder häufen sich in der Zelle an und werden dann mit im Stand der Technik bekannten Methoden, nachdem die Zellen aufgebrochen wurden, isoliert und gegebenenfalls gelöst. Für diese Verfahren werden in der Regel nach bestimmten Fraktionierungs- und Reinigungsvorstufen Detergenzien verwendet, die für die einzelnen, zu isolierenden und zu reinigenden Proteine sehr spezifisch abgestimmt sein müssen. Es ist nach wie vor im Stand der Technik ein Problem, für die gewünschten Proteine die richtigen Detergenzien für die Isolierung und Reinigung zu finden.

Der vorliegenden Erfindung lag die Aufgabe zugrunde, das Glykoprotein 160 von HIV in biologisch aktiver Form sowie Verfahren zu dessen Herstellung bereitzustellen.

Diese Aufgabe wird erfindungsgemäß gelöst durch das Glykoprotein 160 (GP 160) in aufgelockerter, elektronenoptisch sichtbarer, aggregierter, elektrophoretisch nicht mobiler Form (Form II), erhältlich durch Behandeln von in Zellen produziertem, etwa sphäroid aggregiertem, kompaktem, elektronenoptisch sichtbarem, elektrophoretisch nicht mobilem GP 160 (Form I) mit einem nicht-denaturierenden Detergenz und durch das Verfahren zum Isolieren und Reinigen von in Zellen produziertem GP 160, bei dem in einem ersten Reinigungsschritt von dem, in einer ersten aggregierten Form I vorliegenden GP 160 die an der Oberfläche befindlichen Verunreinigungen entfernt werden, GP 160 sodann unter Verwendung eines nicht-denaturierenden Detergenz in eine zweite aggregierte Form II überführt wird und in einem zweiten Reinigungsschritt die in dieser aggregierten Form II zugänglichen Verunreinigungen entfernt werden; gegebenenfalls kann durch Zugabe eines zweiten, nicht-denaturierenden
Detergenz GP 160 in eine monomere Form III übergeführt werden und durch Entfernen des nicht-denaturierenden Detergenz reversibel wieder in die aggregierte Form I oder II übergeführt werden.

Im Folgenden werden unter der "biologischen Aktivität" eines Proteins die Eigenschaften des Proteins verstanden, die es unter physiologischen Bedingungen zeigt, wie beispielsweise die Fähigkeit, an Rezeptoren zu binden oder eine Immunantwort wie das native Protein hervorzurufen.

Unter dem Begriff "amphipatische Proteine" werden im folgenden Proteine verstanden, die hydrophile wie auch hydrophobe Domänen enthalten. Typische Vertreter sind integrale Membranproteine, bei denen die hydrophilen Domänen der wässrigen Umgebung ausgesetzt sind, und die hydrophoben Domänen von der Zellmembran umgeben sind.

Bei Isolierungsverfahren von Proteinen hat sich überraschenderweise gezeigt, daß bei der Verwendung nicht-denaturierender Detergenzien amphipatische Proteine, wie GP 160, nicht in ihrer monomeren Form, sondern in aggregierten Formen vorliegen, die ganz überraschende vorteilhafte Eigenschaften aufweisen. In Abwesenheit eines nicht-denaturierenden Detergenz weisen die aggregierten Formen eine kompakte Struktur auf, die hier als aggregierte Form I bezeichnet werden soll. In dieser Form sind die Partikel bestens geeignet zum Entfernen von Verunreinigungen, die auf der Oberfläche dieser Partikel sitzen. Die kompakte Form der Partikel sichert dabei einen gewissen Schutz vor Denaturierung der aktiven Zentren, seien es enzymatisch aktive Zentren oder Epitope, die Immunreaktionen hervorrufen. Dennoch sind auch die kompakten Partikel in der aggregierten Form I bioaktiv, jedoch elektrophoretisch nicht mobil.

In Anwesenheit eines nicht-denaturierenden Detergenz läßt sich die kompakt sphärische Form I der aggregierten Proteine in eine mehr oder weniger aufgelockerte Form überführen, die im Laufe eines Isolierungs- und Reinigungsverfahrens den Vorteil aufweist, daß Verunreinigungen entfernt werden können, die durch das Auffalten der Form I in die Form II leichter zugänglich werden. In der Form II der aggregierten Proteine verstärken sich weiterhin überraschenderweise die biologischen Aktivitäten der Proteine. Es zeigte sich beispielsweise, daß die Immunogenität der isolierten Proteine ansteigt, wenn die biologische Eigenschaft der isolierten Proteine in der immunisierenden Antigenität der Proteine liegt. Die biologisch aktiven Proteine, oder gegebenenfalls auch Teile dieser Proteine, die eine biologische Aktiviät aufweisen, sind somit insbesondere in der aufgeweiteten Form aktiv und eignen sich in dieser etwa wolkigen Form aus den genannten Gründen besonders gut für die Reinigung der Proteine von Bestandteilen, die in den aggregierten Formen eingeschlossen sind.

Die Proteine in der aggregierten Form II sind ebenfalls elektrophoretisch nicht mobil.

Das besonders Überraschende an dem erfindungsgemäß zur Verfügung gestellten, partikulären, aggregierten Protein GP 160 ist der Umstand, daß die aggregierten Formen I und II reversibel ineinander überführbar sind, in Abhängigkeit von der Abwesenheit oder Anwesenheit eines ersten nicht-denaturierenden Detergenz. Mit dem beschriebenen partikulären aggregierten Protein wird somit ein immens zweckmäßiger Komplex zur Verfügung gestellt, der durch die Reversibiliät des Ineinanderüberführens der verschiedenen Formen I und II dieser Aggregate im Hinblick auf ein erleichtertes Reinigungsverfahren, eine verbesserte Lagerungsmöglichkeit sowie eine erhöhte Wirksamkeit der biologischen Aktivität des isolierten Proteins enorme Vorteile aufweist.

Wenn ein zweites nicht-denaturierendes Detergenz gezielt eingesetzt wird, können Proteine aus der aggregierten Form II in die monomere Form III überführt werden, wobei sodann völlig unerwarteterweise auch dieser Zustand reversibel ist, wenn das zweite, nicht-denaturierende Detergenz entfernt wird. In Abhängigkeit von der Abwesenheit bzw. Anwesenheit der nicht-denaturierenden Detergenzien können aus physiologisch aktiven Zellen zu isolierende Proteine somit gezielt und manipulierbar reversibel in jede gewünschte und für den gerade bezweckten Verfahrensschritt bzw. Verwendungszweck optimale Form überführt werden.

Für die Lagerung bzw. eine dosierte, gegebenenfalls gewünschte, reduzierte biologische Aktivität isolierter Proteine ist eine aggregierte Form I in etwa sphärischer Struktur bevorzugt. Die kugelige Form schützt weiterhin gegebenenfalls bei Reinigungsschritten eventuelle sensitive aktive Zentren (wie die CD4-Rezeptorbindungsstelle, der V₃-Loop, das PND-Epitop, konformationsabhängige Epitope usw. von GP 160) vor eventuell denaturierenden Behandlungsschritten.

Die aggregierte Form II wird je nach Dauer der Behandlung und Konzentration eines nicht-denaturierenden Detergenz eine etwa wolkige Struktur aufweisen. Je nach dem Auffaltungsgrad sind die Proteine weiteren Reinigungsschritten zugänglich bezüglich solcher Verunreinigungen, die beispielsweise von den für die Produktion der Proteine verwendeten Wirtszellen bzw. Vektorsystemen stammen können. Die biologische Aktivität der Proteine ist erhöht.

Sowohl die sphäroide, aggregierte Form I als auch die etwa wolkige, aggregierte Form II können elektronenoptisch sichtbar gemacht werden, wenn das produzierte Einzelprotein ein Molekulargewicht aufweist, das mindestens 10000 Dalton beträgt. Wenn jedoch beispielsweise durch genetische Manipulation nur bestimmte antigene Determinanten exprimiert werden, könnten auch die aggregierten, mehrere Proteine enthaltenden Formen I und II unterhalb der elektronenoptischen Sichtbarkeit liegen.

Vorzugsweise liegen die in den Formen I und II aggregierten, elektrophoretisch nicht mobilen Proteine in einer Reinheit von 80 %, vorzugsweise 98 % vor, d.h. daß die aggregierten Formen I und II die gewünschten Proteine in verhältnismäßig homogener Ansammlung enthalten.

Das Aggregieren der Proteine innerhalb der Zellen tritt bevorzugt dann auf, wenn die Proteine Expressionsprodukte gentechnisch manipulierter Zellen sind und in diesen Zellen wiederum insbesondere dann, wenn die Produkte mit bekannten Methoden in diesen Zellen überproduziert werden. Die Überproduktion kann erfolgen, indem entweder mehrere Vektoren, die das Gen enthalten, die das gewünschte Protein exprimieren, vorliegen, oder wenn besonders starke Promotoren vor die entsprechenden Gene geschaltet sind. Soweit es sich bei den exprimierten Proteinen dann um solche handelt, die in der Zelle löslich sind, aggregieren sie sich zu den beschriebenen sphärischen Partikeln.

Die von den genetisch manipulierten Wirtszellen exprimierten Proteine weisen bevorzugt eine antigene Funktion auf. Diese Proteine sind bevorzugte Projekte für die genmanipulierte Expression von Proteinen, wenn Lebendimpfstoffe zur Verfügung gestellt werden sollen, bzw. wenn durch die Antigene eine entsprechende aktive Immunisierung erreicht werden soll. Es handelt sich dabei insbesondere um Hüllproteine pathogener Viren, seien es humanpathogene oder tierpathogene Viren.

Bei dem erfindungsgemäßen Protein handelt es sich um das Glykoprotein GP 160 des HIV. Die Gene für die Expression der jeweiligen Proteine können zum Beispiel durch einen Vaccinia-Virusvektor in einem Säugetierzellsystem exprimiert werden. Das Verfahren, wie spezifische Gene in Vaccinia-Virusvektoren insertiert werden, ist bei Mackett et al. (J. Virol, 49, S. 857 - 846, 1984) beschrieben. In an sich im Stand der Technik derzeit bekannter Weise wird das fremde Gen zuerst in einen Plasmidvektor stromabwärts von einem Vaccinia-Transkriptions-Kontrollelement (7,5 K-Promotor) insertiert. Dieses chimäre Gen in dem rekombinanten Plasmid wird durch Vaccinia-Sequenzen flankiert, die einen nicht-essentiellen Bereich des Genoms darstellen (z.B. ein Genabschnitt, der für das virale Thymidinkinasegen (TK) codiert). Das Plasmid wird sodann in Zellen eingeführt, die vorher mit einem Wildtyp-Vacciniavirus (Stamm WR) infiziert wurden. Die Rekombination geschieht dann in der TK-Region, die sowohl für die virale DNA als auch für das Plasmid homolog ist und die Insertion des chimären Gens in das Genom des Vaccinia-Virus erlaubt. Das auf diese Weise erhaltene rekombinante Virus weist den Phänotyp TK⁻ auf, ist also nicht mehr in der Lage, die Thymidinkinase zu produzieren und wächst in Selektivmedium, das 5-Brom-deoxyuridin enthält. Auf diese Weise können rekombinante Vaccinia-Viren hergestellt werden, die jeweils Gene tragen, die Proteine, gegebenenfalls in Überproduktion, produzieren, die sich sodann in den Säugetierzellen zu den geschilderten, eine Vielzahl der einzelnen Proteine enthaltenden aggregierten Formen zusammenlagern. Ähnliche Expressionssysteme sind für andere antigene Proteine bekannt. Beispielsweise sind einige Vaccinia-Virus-Rekombinanten konstruiert worden, die für die Oberflächenantigene des Hepatitis-B-Virus codieren. Zu diesem Zweck wurden die Hepatitis-B-Virus-Oberflächen-Antigene unter den regulativen Mechanismen des Vaccinia-Virus exprimiert, wobei eine Anzahl von vaccinia-spezifischen Promotoren verwendet wurden mit dem Ziel, das Expressionsniveau für die fremden Gene zu erhöhen (Übersicht in "Vaccinia Viruses as Vectors für Vaccine Antigens", Hrsg. Gerald v. Quinnan, Elsevier Science Publishing (1985)). Es wurden weiterhin Vaccinia-Virus-Rekombinante hergestellt, die mehr als ein Hepatitis B-Virusgen exprimieren.

Ganz allgemein wird an dieser Stelle bezüglich der Herstellung rekombinanter Expressionssysteme zum Zwecke des gentechnischen Produzierens von gewünschten Proteine auf Winnacker, E.L. (Gene und Klone, eine Einführung in die Gentechnologie, VCH-Verlagsgesellschaft mbH, Weinheim, 1984) verwiesen.

Die in partikulären Formen angesammelten Proteine können vorzugsweise an eine weitere Substanz, insbesondere ein hochmolekulares Protein durch Konjugation gebunden werden, wodurch eine Verbesserung bezüglich der Stabilität, Aktivität, in vivo-Wiedergewinnung und biologischen Halbwertzeit der Proteine erreicht wird. Ein Beispiel für eine derartige Konjugation von Proteinen mit weiteren hochmolekularen Substanzen stellt das Konjugat aus Blutgerinnungsfaktor VIII und dem von Willebrands-Faktors dar.

Die partikulär aggregierten Proteine liegen bevorzugt in eukaryontischen Zellen, insbesondere tierischen oder humanen Zellen vor.

Besonders bevorzugt werden dabei die gewünschten Proteine in primären Zellkulturen oder aus Zellinien gewonnenen Verozellen, CHO-Zellen oder primären Hühnerembryofibroblastenzellen exprimiert. Die genannten Wirtszellen sind als Expressionssystem bevorzugt, da hier bereits die nach der Translation erfolgenden Modifikationen in einer Weise vorliegen, wie sie bei den zu verwendenden Endproduktion gewünscht sind, wie beispielsweise die Glykosilierung der exprimierten Proteine. Diese glykosilierten Proteine lagern sich sodann zu der partikulären Form zusammen, die die beschriebenen sphärischen Formen I bzw. wolkigen Formen II nach Behandlung mit einem ersten, nicht-denaturierenden Detergenz annehmen können, und nach Behandlung mit einem zweiten nicht-denaturierenden Detergenz in eine dritte Form III überführt werden können.

Verozellen sind besonders bevorzugt für die Herstellung des Glykoproteins 160 des HIV, wie es oben beschrieben wurde. Dieses Zellsystem wurde ausgewählt, da es eine verhältnismäßig schnelle Wachstumsrate hat und für die Herstellung menschlicher Impfstoffe bereits gut adaptiert ist. Die Infektion von Verozellen mit einem rekombinanten Vacciniavirus, das ein Gen enthält, das das gewünschte, nach dem vorliegenden, erfinderischen Verfahren zu isolierende Protein exprimiert, resultiert in normaler Synthese des gewünschten Proteins, entsprechender Glykosilierung und Prozessierung. Durch die Verwendung eines Doppel-Infektionssystems mit zwei Rekombinanten kann die Menge des gewünschten Proteins erheblich gesteigert werden. Dieses System beinhaltet eine Rekombinante, die die Polymerase des Phagen T7 unter der Steuerung des P7.5-Promotors von Vaccinia exprimiert. Diese Rekombinante wird zusammen mit einer Rekombinanten infiziert, die den T7-Promotor und das Glykoprotein 160 Gen des HIV enthält. Der T7-Promotor ist ein sehr starker Promotor und sichert ein hohes Niveau der Expression.

Die beschriebenen Systeme sind als bevorzugte Systeme zu verstehen. Eine allgemeine Übersicht über die vielfältigen Möglichkeiten, bestimmte Vektoren für die Rekombination gewünschter Gene zu verwenden und diese Vektoren in gewünschten Zellen zu vermehren und die durch die Fremdgene exprimierten Proteine herzustellen findet sich beispielsweise in Genetic Engeneering 3, Academic Press, Edited by Robert Williamson, (1982) oder in Spektrum der Wissenschaft, Industrielle Mikrobiologie (1985).

Die nicht-denaturierenden Detergenzien, deren Anwesenheit oder Abwesenheit das Vorliegen der aus den physiologisch aktiven Zellen isolierten Proteine in voneinander verschiedenen aggregierten Formen oder in monomerer Form der Proteine wesentlich beeinflußt, können ionische, nichtionische oder zwitterionische Detergenzien sein. Es ist in der Proteinchemie bzw. bei den Protein-Reinigungsverfahren ein bekanntes Problem, daß die Wahl des Detergenz für die Wiedergewinnung des gewünschten Proteins in einerseits hochgereinigter Form, andererseits in biologisch aktivem Zustand jedesmal eine Herausforderung darstellt. Es hat sich nun überraschenderweise herausgestellt, daß durch die Verwendung von nicht-denaturierenden Detergenzien, bestimmten ionischen, nicht-ionischen oder zwitterionischen Charakters die oben genannten Forderungen an ein Protein-Reinigungsverfahren voll erfüllt werden können, wobei zusätzlich die unerwartete Konformationsänderung zusammengeballter Proteine, wie sie in den diese Proteine produzierenden Zellen vorliegen, die genannten Forderungen in verblüffender Weise optimal erfüllen. Wie bereits erwähnt, wird insbesondere in der aggregierten Form II, der aufgelockerten oder wolkigen Konformation, die dem einzelnen Protein innewohnende biologische Aktivität, sei sie enzymatischer oder antigener Natur, deutlich verstärkt.

Ein bevorzugtes, nicht-ionisches Detergenz ist das Octylglucosid oder eins der Derivate dieses Detergenz, vorzugsweise in Konzentrationen von 0,25 - 5%, insbesondere 1%.

Ebenso bevorzugt ist die Verwendung der Salze der Gallensäure sowie eines seiner Derivate, bevorzugt das Detergenz Desoxycholat (DOC), das insbesondere geeignet ist im Zusammenhang mit der Isolierung und Reinigung der sphärischen Konformation des Glykoproteins 160 des HIV. DOC wird zur Isolierung vorzugsweise in Konzentrationen von 0,25 - 5%, insbesondere 1%, als auch bei der Applikation des GP 160, beispielsweise in Verbindung mit Aluminiumhydroxid, verwendet.

Das nicht-denaturierende Detergenz, das für die Überführung der aggregierten Form II in die monomere Form III bevorzugt zu verwenden ist, ist ein zwitterionisches Detergenz der Zwittergent-Reihe, ebenfalls bevorzugt in Konzentrationen von 0,25 - 5%, insbesondere 1%.

GP 160 in der aggregierten Form I eignet sich, wie bereits erwähnt, besonders gut für die stabile Lagerung. Es kann jedoch auch für die gewünschten biochemischen Umsetzungen oder Reaktionen verwendet werden, da auch das in der aggregierten Form I zusammengeballte Protein biologische Aktivität aufweist.

Die Verwendung von GP 160 in der aggregierten Form II geschieht bevorzugt zum Verstärken der biologischen Funktion des Proteins.

Die Form II des Proteins eignet sich für eine therapeutische, prophylaktische oder diagnostische Anwendung, wie auch die oben beschriebenen konjugierten Formen mit weiteren hochmolekularen Proteinen.

Eine weitere Verwendung des aggregierten Proteins in Form II liegt in der Substitutionsbehandlung, der Gewinnung von Impfstoffen oder monoklonalen Antikörpern sowie zur Gewinnung von Substanzen zum qualitativen Nachweis und quantitativen Bestimmen solcher Substanzen in Körperflüssigkeiten.

Neben den aus einer Anzahl von Proteinen bestehenden Partikeln, die in Abhängigkeit von Anwesenheit oder Abwesenheit eines nicht-denaturierenden Detergenz in morphologisch verschiedenen Konformationen vorliegen, in die sie reversibel ineinander überführt werden können, bezieht sich die vorliegende Erfindung auf ein Verfahren zum Isolieren und Reinigen von GP 160, das in Zellen, bevorzugt gentechnisch manipulierten Zellen, produziert wird, wie oben beschrieben.

Die immensen Vorteile dieses Verfahrens sind im wesentlichen oben bereits diskutiert und liegen in der reversiblen Überführbarkeit der verschiedenen aggregierten Formen des Proteins ineinander.

Die etwa sphärische Form I erlaubt durch die kompakte Anordnung der Proteine beispielsweise eine erleichterte erste Reinigung von beispielsweise Medienbestandteilen von der Oberfläche der quasi kugeligen Proteinzusammenballung. Wenn sodann der zweite Reinigungsschritt von Verunreinigungen, die in der aggregierten Form II durch die Auffaltung besonders leicht zugänglich sind, abgelaufen ist, kann durch Entzug des nicht-denaturierenden Detergenz reversibel die aggregierte Form I wieder hergestellt werden, in der beispielsweise aktive Zentren des Proteins besonders gut geschützt im Inneren der sphärischen Proteinanordnungen liegen, so daß eine besonders gute Lagerung des Proteins in der aggregierten Form I möglich ist. Die aggregierte Form II hat, wie bereits erwähnt, neben den Vorteilen hinsichtlich der erleichterten Reinigung von Zellbestandteilen der Wirtszelle, in denen das Protein exprimiert wurde, den weiteren Vorteil, daß die biologische Aktivität des Proteins durch die räumliche Nähe vieler aktiver Zentren durch den speziellen aggregierten Zustand des Proteins wesentlich verstärkt werden kann.

Je nach Versuchsanordnung werden Zellen oder Zellmembranen abzentrifugiert und mit einer Pufferlösung, die ein nicht-denaturierendes Detergenz sowie ein Proteasehemmsystem enthält, extrahiert, oder, wenn die gewünschte, hochmolekulare Substanz von den Zellen in den Überstand abgegeben wird, wird dieser Überstand mit dem nicht-denaturierenden Detergenz und dem Proteasehemmsystem versetzt, wobei das nicht-denaturierende Detergenz beispielsweise Desoxycholat (DOC), in Konzentrationen von vorzugsweise 0,25 - 5%, insbesondere jedoch 1%, vorliegt.

Die so erhaltenen Lösungen werden, wenn es sich bei der zu isolierenden und reinigenden Substanz um ein Glykoprotein handelt, mittels Lektinen, die an eine Festmatrix gekoppelt sind, ankonzentriert und von nicht kohlehydrathaltigen Verunreinigungen abgetrennt. Das verwendete, nicht-denaturierende Detergenz hat bei diesem Schritt zusätzlich zur Eigenschaft, die Substanz in die Form II zu überführen, das Vermögen, eine unspezifische Adsorption zu verhindern. Das Eluieren erfolgt mit Glukosiden.

Ein weiterer, wesentlicher Reinigungsschritt kann durch spezielle Bindung der vorgereinigten, hochmolekularen Substanz an eine Immunadsorptionssäule erreicht werden. Nach ausreichendem Waschen mit einem Puffer wird mit einem chaotropen Agenz, beispielsweise Harnstoff oder KSCN (2 - 8 M, vorzugsweise 3 M) eluiert. Durch anschließende Dialyse gegen einen Puffer wird in die partikuläre Form I überführt. Ist es erforderlich, wird die hochmolekulare Substanz durch ein bestimmtes, zwitterionisches Detergenz in die monomere Form III überführt und durch Ionenaustauschchromatographie gereinigt. Durch Dialyse gegen einen detergenzienfreien Puffer werden aus der monomeren Form III die aggregierten Formen II oder I wieder hergestellt.

Die verschiedenen aggregierten Formen der in den beschriebenen Zellen produzierten Proteine können elektronenmikroskopisch sichtbar gemacht werden.

Die Erfindung wird im folgenden detailliert anhand der Figuren und der Beispiele erläutert.

### Es zeigen:

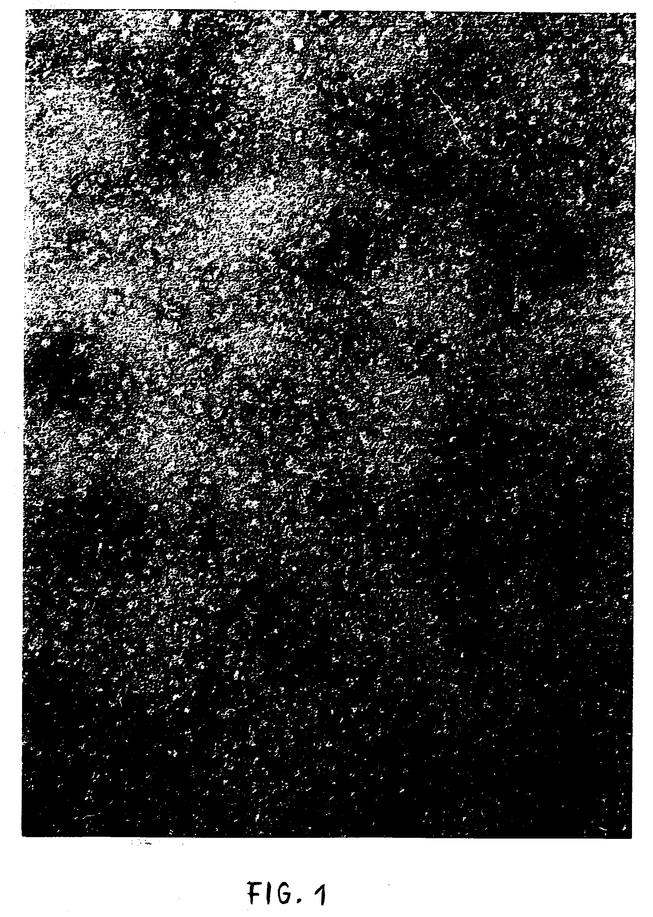

Fig. 1: Eine elektronenmikroskopische Aufnahme des Glykoproteins 160 in Tris-Puffer in 278 640-facher Vergrößerung.

Die Proteine liegen in der kompakten, sphärischen aggregierten Form I vor.

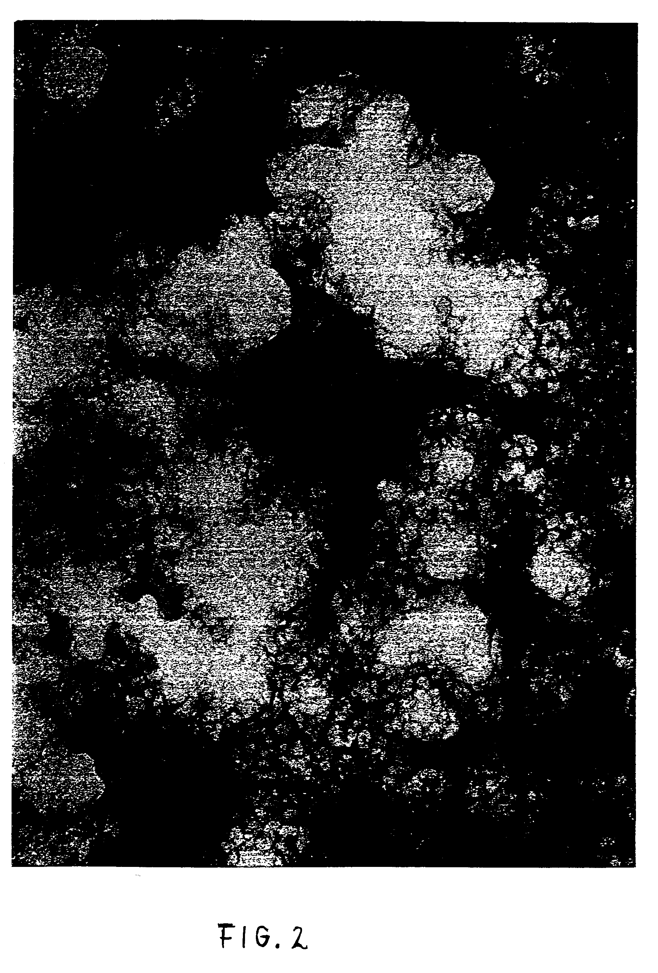

Fig. 2: Eine elektronenmikroskopische Aufnahme des Glykoproteins 160 des HIV in einem Puffer, der 1 % DOC enthält.

Die Proteine liegen in der lockeren, wolkigen aggregierten Form II vor.

### Beispiel 1

Isolieren und Reinigen des Glykoprotein 160 des HIV.

Das Isolieren und Reinigen des Glykoprotein 160 des HIV erfolgt nach folgendem Schema:
1. Gentechnisch manipulierte Verozellen, die das Glykoprotein 160 des HIV produzieren, werden bei einer gewünschten Dichte zentrifugiert.
2. Das durch Zentrifugation gewonnene Pellet wird in TBS pH 7,4 plus 1 mM CuSO₄ plus 0,5 mM ZnCl₂ resuspendiert.
3. Das resuspendierte Pellet wird einer Extraktion mit 50 mM Tris-HCl pH 8,3, 1 % DOC, 1 mM CuSO₄ und 0,5 mM ZnCl₂ unterworfen. Die Suspension wird 30 Minuten lang bei 25°C durch Zentrifugieren geklärt.
4. Der Überstand wird einer Lentil-Lectin-Chromatographie unterworfen, indem man die gewünschten Proteine an eine Säule adsorbiert und mit DOC-Puffer wäscht. Die Proteine werden mit 5 % Methylglucosid in einem Waschpuffer eluiert.
5. Das Eluat aus der Lectin-Säule wird in Tween-Puffer verdünnt. Sodann erfolgt eine Adsorption an eine Immunaffinitäts-Chromatographiesäule, die mit TBS-Tween und anschließend mit TBS gewaschen wird. Es folgt eine DNase- und RNase-Behandlung. Es wird mit 3M KSCN eluiert und gegen detergenzienfreien Puffer dialysiert.
6. Das dialysierte Eluat wird mit 1% Zwittergent und 5% Betain justiert und sodann an eine Mono-Q-Matrix als Anionenaustauscher adsorbiert und sodann mit einem KSCN-Gradienten eluiert und gegen einen Puffer dialysiert.
7. Es erfolgt eine zweite Lentil-Lectin-Chromatographie durch Adsorbieren und Waschen mit detergenzienfreien Puffer. Das Eluieren erfolgt mit 5 %igem Methylglucosid, gefolgt von einer Dialyse gegen TBS. Mit diesem Verfahrensschritt erfolgt eine Konzentrierung der Proteine.

### Beispiel 2

Mit dem wie im Beispiel 1 beschriebenen Verfahren gereinigten Glykoprotein (GP) 160 des HIV wurde ein Potency-Test durchgeführt, der die immunogene Wirksamkeit des Glykoproteins 160 in kompakter, sphärischer Form I, sowie in aufgelockerter, wolkiger Form II, zeigt.

Pro Vaccine werden 5 Verdünnungen mit 10 µg, 2,5 µg, 0,625 µg, 0,158 µg sowie 0,04 µg GP 160/ml hergestellt. Pro Verdünnung werden 10 BALBc Mäuse subcutan immunisiert; pro Vaccinenart werden demnach 50 Tiere benötigt. Beim vorliegenden Versuch wurden vier verschiedene Adsorptionsmethoden getestet.
1. GP 160 + 0,2 % Al(OH)₃
2. [GP 160 + 0,25 % DOC] + 0,2 % Al(OH)₃
3. [GP 160 + 0,25 % DOC] + [0,2 % Al(OH)₃ + 0,25 % DOC]
4. [GP 160 + 0,25 % DOC] + [0,2 % Al(OH)₃ gewaschen]

Alle Tiere wurden mit 1 ml subcutan immunisiert und nach sechs Wochen einzeln entblutet. Das Serum jedes einzelnen Tieres wurde mittels Anti-HIV ELISA (SORIN) auf das Vorhandensein von GP 160 Antikörpern untersucht, wobei für jede Vaccinenart die effektive Dosis 50 von GP 160 anhand der reagierenden Tiere mittels der bekannten Spearman-Kaerber-Methode berechnet wurde.

### Ergebnisse

1. I - V 10µg GP 160/ml + 0,2 % Al(OH)₃ s.c.

| | | reag. Tiere | Titer max. |
|---|---|---|---|
| I | conz. | 1 | 100 |
| II | 1: 4 | 1 | 10 |
| III | 1: 16 | 0 | / |
| IV | 1: 64 | 1 | 10 |
| V | 1:256 | 1 | 10 |
| ED₅₀: 10 µg/ml GP 160 | | | |

2. VI - X [10 µg GP 160/ml + DOC] + 0,2 % Al(OH)₃ s.c.

| | | reag. Tiere | Titer max. |
|---|---|---|---|
| VI | conz. | 7 | 1000 |
| VII | 1: 4 | 5 | 200 |
| VIII | 1: 16 | 1 | 10 |
| IX | 1: 64 | 0 | / |
| X | 1:256 | 0 | / |
| ED₅₀: 3,3 µg/ml GP 160 | | | |

3. XI - XV [10 µg GP 160/ml + DOC] + [Al(OH)₃ + DOC] s.c.

| | | reag. Tiere | Titer max. |
|---|---|---|---|
| XI | conz. | 7 | 800 |
| XII | 1: 4 | 5 | 1000 |
| XIII | 1: 16 | 6 | 100 |
| XIV | 1: 64 | 2 | 100 |
| XV | 1:256 | 1 | 100 |
| ED₅₀: 1,09 µg/ml GP 160 | | | |

4. XVI - XX [10µg GP 160/ml + DOC] + Al(OH)₃ gewaschen s.c.

| | | reag. Tiere | Titer max. |
|---|---|---|---|
| XVI | conz. | 0 | / |
| XVII | 1: 4 | 1 | 10 |
| XVIII | 1: 16 | 0 | / |
| XIX | 1: 64 | 0 | / |
| XX | 1:256 | 0 | / |
| ED₅₀: 10 µg/ml GP 160 | | | |

Das Ergebnis des Potency Tests des Glykoproteins 160 des HIV zeigt die unterschiedliche Immunogenität des Glykoproteins 160 in Abhängigkeit der aggregierten Form. Der ED₅₀-Wert, der diejenige Menge der verwendeten Substanz bezeichnet, bei der 50% der geimpften Tiere serokonvertieren, verringert sich erheblich, wenn das Detergenz DOC anwesend ist. In Anwesenheit des Detergenz DOC liegt das Glykoprotein 160 in der aggregierten Form II vor, in der die antigenen Epitope einerseits freiliegen, andererseits jedoch räumlich zusammenhängen, so daß eine verstärkte Immunantwort erfolgt.

## Patentansprüche (Patentansprüche für folgende(n) Vertragsstaat(en): AT, BE, CH, DE, FR, GB, IT, LI, LU, NL, SE)

1. GP 160 in aufgelockerter, elektronenoptisch sichtbarer, aggregierter, elektrophoretisch nicht mobiler Form (Form II), erhältlich durch Behandeln von in Zellen produziertem, etwa sphäroid aggregiertem, kompaktem, elektronenoptisch sichtbarem, elektrophoretisch nicht mobilem GP 160 (Form I) mit einem nicht-denaturierenden Detergenz.

2. GP 160 nach Anspruch 1, **dadurch gekennzeichnet,** daß es in einer mindestens 80%igen, vorzugsweise 98%igen Reinheit vorliegt.

3. GP 160 nach einem der Ansprüche 1 oder 2, **dadurch gekennzeichnet,** daß es ein Expressionsprodukt gentechnisch manipulierter Zellen, insbesondere solcher ist, die die Proteine überproduzieren.

4. GP 160 nach Anspruch 3, **dadurch gekennzeichnet,** daß es eine antigene Funktion aufweist.

5. GP 160 nach mindestens einem der vorhergehenden Ansprüche, **dadurch gekennzeichnet,** daß die Zellen eukaryontische, bevorzugt tierische oder humane, Zellen sind.

6. GP 160 nach Anspruch 5, **dadurch gekennzeichnet,** daß die Zellen aus primären Zellkulturen oder aus Zellinien gewonnen werden, insbesondere Verozellen, CHO-Zellen oder primäre Hühnerembryofibroblastenzellen.

7. GP 160 nach mindestens einem der vorhergehenden Ansprüche, **dadurch gekennzeichnet,** daß die Detergenzien bestimmte ionische, nicht-ionische oder zwitterionische Detergenzien sind.

8. GP 160 nach Anspruch 7, **dadurch gekennzeichnet,** daß als erstes, nicht-ionisches Detergenz zur Überführung von Form I in Form II Octylglucosid oder ein Derivat von diesem, vorzugsweise in einer Konzentration von 0,25 bis 5%, insbesondere 1%, und als zweites, zwitterionisches Detergenz zur Überführung von Form II in die monomere Form (Form III) eins der Zwittergent-Reihe, vorzugsweise in einer Konzentration von 0,25 bis 5%, insbesondere 1%, verwendet wird.

9. GP 160 nach Anspruch 7, **dadurch gekennzeichnet,** daß das Detergenz ein Salz der Gallensäure ist oder ein Derivat von diesem, bevorzugt Desoxycholat (DOC), vorzugsweise in einer Konzentration von 0,25 bis 5%, insbesondere 1%.

10. Verwendung von GP 160 nach einem der vorhergehenden Ansprüche in seiner aggregierten Form I zum stabilen Lagern.

11. Verwendung von GP 160 nach einem der vorhergehenden Ansprüche in seiner aggregierten Form II zum Verstärken der biologischen Funktion.

12. GP 160 in Form II zur therapeutischen, prophylaktischen oder diagnostischen Anwendung.

13. Verwendung von GP 160 in Form II zum Gewinnen von Impfstoffen.

14. Verwendung von GP 160 in Form II zur Gewinnung von Substanzen zum qualitativen Nachweis oder quantitativen Bestimmen der genannten Substanzen in Körperflüssigkeiten.

15. Verfahren zum Isolieren und Reinigen von in Zellen produziertem GP 160, **dadurch gekennzeichnet,** daß in einem ersten Reinigungsschritt von dem, in einer ersten aggregierten Form I vorliegenden GP 160 die an der Oberfläche befindlichen Verunreinigungen entfernt werden, GP 160 sodann unter Verwendung eines nicht-denaturierenden Detergenz in eine zweite aggregierte Form II überführt wird und in einem zweiten Reinigungsschritt die in dieser aggregierten Form II zugänglichen Verunreinigungen entfernt werden und gegebenenfalls durch Zugabe eines zweiten, nicht-denaturierenden Detergenz GP 160 in eine monomere Form III übergeführt wird und durch Entfernen des nicht-denaturierenden Detergenz reversibel wieder in die aggregierte Form I oder II überführbar ist.

16. Verfahren nach Anspruch 15, **dadurch gekennzeichnet,** daß zu Reinigungszwecken das reversible Überführen der aggregierten Formen I, II und III mehrfach durchgeführt wird.

17. Verfahren nach Anspruch 15, **dadurch gekennzeichnet,** daß das GP 160 ein Expressionsprodukt gentechnisch manipulierter Zellen, insbesondere solcher, die Proteine überproduzieren, ist.

18. Verfahren nach mindestens einem der Ansprüche 15 oder 17, **dadurch gekennzeichnet,** daß die Zellen eukaryontische, bevorzugt tierische oder humane Zellen sind.

19. Verfahren nach Anspruch 18, **dadurch gekennzeichnet,** daß die Zellen aus primären Zellkulturen oder aus Zelllinien gewonnen werden, insbesondere Verozellen, CHO-Zellen oder primäre Hühnerembryofibroblastenzellen.

20. Verfahren nach Anspruch 15, **dadurch gekennzeichnet,** daß die Detergenzien bestimmte ionische, nicht-ionische oder zwitterionische Detergenzien sind.

21. Verfahren nach Anspruch 20, **dadurch gekennzeichnet,** daß als erstes, nicht-ionisches Detergenz Octylglucosid oder ein Derivat von diesem, vorzugsweise in einer Konzentration von 0.25 bis 5%, insbesondere 1%, und als zweites, zwitterionisches, Detergenz eins der Zwittergent-Reihe, vorzugsweise in einer Konzentration von 0,25 bis 5%, insbesondere 1%, verwendet wird.

22. Verfahren nach Anspruch 20, **dadurch gekennzeichnet,** daß das Detergenz ein Salz der Gallensäure oder ein Derivat von diesem ist, bevorzugt Deoxycholat (DOC), vorzugsweise in einer Konzentration von 0,25 bis 5%, insbesondere 1%.

## Patentansprüche (Patentansprüche für folgende(n) Vertragsstaat(en): ES)

1. Verfahren zum Herstellen von GP 160 in aufgelockerter, elektronenoptisch sichtbarer, aggregierter, elektrophoretisch nicht mobiler Form (Form II), wobei in Zellen produziertes, etwas sphäroid aggregiertes, kompaktes, elektronenoptisch sichtbares, elektrophoretisch nicht mobiles GP 160 (Form I) mit einem nicht-denaturierenden Detergenz behandelt wird.

2. Verfahren nach Anspruch 1, **dadurch gekennzeichnet,** daß es zu einem GP 160 mit einer mindestens 80%igen, vorzugsweise 98%igen Reinheit führt.

3. Verfahren nach einem der Ansprüche 1 oder 2, **dadurch gekennzeichnet,** daß das GP 160 ein Expressionsprodukt gentechnisch manipulierter Zellen, insbesondere solcher ist, die die Proteine überproduzieren.

4. Verfahren nach Anspruch 3, **dadurch gekennzeichnet,** daß das GP 160 eine antigene Funktion aufweist.

5. Verfahren nach mindestens einem der vorhergehenden Ansprüche, **dadurch gekennzeichnet,** daß die Zellen eukaryontische, bevorzugt tierische oder humane Zellen sind.

6. Verfahren nach Anspruch 5, **dadurch gekennzeichnet,** daß die Zellen aus primären Zellkulturen oder aus Zellinien gewonnen werden, insbesondere Verozellen, CHO-Zellen oder primäre Hühnerembryofibroblastenzellen.

7. Verfahren nach mindestens einem der vorhergehenden Ansprüche, **dadurch gekennzeichnet,** daß die Detergenzien bestimmte ionische, nicht-ionische oder zwitterionische Detergenzien sind.

8. Verfahren nach Anspruch 7, **dadurch gekennzeichnet,** daß als erstes, nicht-ionisches Detergenz zur Überführung von Form I in Form II Octylglucosid oder ein Derivat von diesem, vorzugsweise in einer Konzentration von 0,25 bis 5%, insbesondere 1%, und als zweites, zwitterionisches Detergenz zur Überführung von Form II in die monomere Form (Form III) eins der Zwittergent-Reihe, vorzugsweise in einer Konzentration von 0,25 bis 5%, insbesondere 1%, verwendet wird.

9. Verfahren nach Anspruch 7, **dadurch gekennzeichnet,** daß das Detergenz ein Salz der Gallensäure ist oder ein Derivat von diesem, bevorzugt Desoxycholat (DOC), vorzugsweise in einer Konzentration von 0,25 bis 5%, insbesondere 1%.

10. Verwendung von GP 160 nach einem der vorhergehenden Ansprüche in seiner aggregierten Form I zum stabilen Lagern.

11. Verwendung von GP 160 nach einem der vorhergehenden Ansprüche in seiner aggregierten Form II zum Verstärken der biologischen Funktion.

12. Verfahren zum Herstellen einer Zusammensetzung zur therapeutischen, prophylaktischen oder diagnostischen Anwendung, **dadurch gekennzeichnet,** daß GP 160 in Form II in an sich üblicher Weise mit Zusatzmitteln, Trägerstoffen oder Verdünnungsmitteln kombiniert wird.

13. Verwendung von GP 160 in Form II zum Gewinnen von Impfstoffen.

14. Verwendung von GP 160 in Form II zur Gewinnung von Substanzen zum qualitativen Nachweis oder quantitativen Bestimmen der genannten Substanzen in Körperflüssigkeiten.

15. Verfahren zum Isolieren und Reinigen von in Zellen produziertem GP 160, **dadurch gekennzeichnet,** daß in einem ersten Reinigungsschritt von dem, in einer ersten aggregierten Form I vorliegenden GP 160 die an der Oberfläche befindlichen Verunreinigungen entfernt werden, GP 160 sodann unter Verwendung eines nicht-denaturierenden Detergenz in eine zweite aggregierte Form II überführt wird und in einem zweiten Reinigungsschritt die in dieser aggregierten Form II zugänglichen Verunreinigungen entfernt werden und gegebenenfalls durch Zugabe eines zweiten, nicht-denaturierenden Detergenz GP 160 in eine monomere Form III übergeführt wird und durch Entfernen des nicht-denaturierenden Detergenz reversibel wieder in die aggregierte Form I oder II überführbar ist.

16. Verfahren nach Anspruch 15, **dadurch gekennzeichnet,** daß zu Reinigungszwecken das reversible Überführen der aggregierten Formen I, II und III mehrfach durchgeführt wird.

17. Verfahren nach Anspruch 15, **dadurch gekennzeichnet,** daß das GP 160 ein Expressionsprodukt gentechnisch manipulierter Zellen, insbesondere solcher, die Proteine überproduzieren, ist.

18. Verfahren nach mindestens einem der Ansprüche 15 oder 17, **dadurch gekennzeichnet,** daß die Zellen eukaryontische, bevorzugt tierische oder humane Zellen sind.

19. Verfahren nach Anspruch 18, **dadurch gekennzeichnet,** daß die Zellen aus primären Zellkulturen oder aus Zelllinien gewonnen werden, insbesondere Verozellen, CHO-Zellen oder primäre Hühnerembryofibroblastenzellen.

20. Verfahren nach Anspruch 15, **dadurch gekennzeichnet,** daß die Detergenzien bestimmte ionische, nicht-ionische oder zwitterionische Detergenzien sind.

21. Verfahren nach Anspruch 20, **dadurch gekennzeichnet,** daß als erstes, nicht-ionisches Detergenz Octylglucosid oder ein Derivat von diesem, vorzugsweise in einer Konzentration von 0.25 bis 5%, insbesondere 1%, und als zweites, zwitterionisches, Detergenz eins der Zwittergent-Reihe, vorzugsweise in einer Konzentration von 0,25 bis 5%, insbesondere 1%, verwendet wird.

22. Verfahren nach Anspruch 20, **dadurch gekennzeichnet,** daß das Detergenz ein Salz der Gallensäure oder ein Derivat von diesem ist, bevorzugt Deoxycholat (DOC), vorzugsweise in einer Konzentration von 0,25 bis 5%, insbesondere 1%.

## Patentansprüche (Patentansprüche für folgende(n) Vertragsstaat(en): GR)

1. GP 160 in aufgelockerter, elektronenoptisch sichtbarer, aggregierter, elektrophoretisch nicht mobiler Form (Form II), erhältlich durch Behandeln von in Zellen produziertem, etwa sphäroid aggregiertem, kompaktem, elektronenoptisch sichtbarem, elektrophoretisch nicht mobilem GP 160 (Form I) mit einem nicht-denaturierenden Detergenz.

2. GP 160 nach Anspruch 1, **dadurch gekennzeichnet,** daß es in einer mindestens 80%igen, vorzugsweise 98%igen Reinheit vorliegt.

3. GP 160 nach einem der Ansprüche 1 oder 2, **dadurch gekennzeichnet,** daß es ein Expressionsprodukt gentechnisch manipulierter Zellen, insbesondere solcher ist, die die Proteine überproduzieren.

4. GP 160 nach Anspruch 3, **dadurch gekennzeichnet,** daß es eine antigene Funktion aufweist.

5. GP 160 nach mindestens einem der vorhergehenden Ansprüche, **dadurch gekennzeichnet,** daß die Zellen eukaryontische, bevorzugt tierische oder humane, Zellen sind.

6. GP 160 nach Anspruch 5, **dadurch gekennzeichnet,** daß die Zellen aus primären Zellkulturen oder aus Zellinien gewonnen werden, insbesondere Verozellen, CHO-Zellen oder primäre Hühnerembryofibroblastenzellen.

7. GP 160 nach mindestens einem der vorhergehenden Ansprüche, **dadurch gekennzeichnet,** daß die Detergenzien bestimmte ionische, nicht-ionische oder zwitterionische Detergenzien sind.

8. GP 160 nach Anspruch 7, **dadurch gekennzeichnet,** daß als erstes, nicht-ionisches Detergenz zur Überführung von Form I in Form II Octylglucosid oder ein Derivat von diesem, vorzugsweise in einer Konzentration von 0,25 bis 5%, insbesondere 1%, und als zweites, zwitterionisches Detergenz zur Überführung von Form II in die monomere Form (Form III) eins der Zwittergent-Reihe, vorzugsweise in einer Konzentration von 0,25 bis 5%, insbesondere 1%, verwendet wird.

9. GP 160 nach Anspruch 7, **dadurch gekennzeichnet,** daß das Detergenz ein Salz der Gallensäure ist oder ein Derivat von diesem, bevorzugt Desoxycholat (DOC), vorzugsweise in einer Konzentration von 0,25 bis 5%, insbesondere 1%.

10. Verwendung von GP 160 nach einem der vorhergehenden Ansprüche in seiner aggregierten Form I zum stabilen Lagern.

11. Verwendung von GP 160 nach einem der vorhergehenden Ansprüche in seiner aggregierten Form II zum Verstärken der biologischen Funktion.

12. Verfahren zum Herstellen einer Zusammensetzung zur therapeutischen, prophylaktischen oder diagnostischen Anwendung, **dadurch gekennzeichnet,** daß GP 160 in Form II in an sich üblicher Weise mit Zusatzmitteln, Trägerstoffen oder Verdünnungsmitteln kombiniert wird.

13. Verwendung von GP 160 in Form II zum Gewinnen von Impfstoffen.

14. Verwendung von GP 160 in Form II zur Gewinnung von Substanzen zum qualitativen Nachweis oder quantitativen Bestimmen der genannten Substanzen in Körperflüssigkeiten.

15. Verfahren zum Isolieren und Reinigen von in Zellen produziertem GP 160, **dadurch gekennzeichnet,** daß in einem ersten Reinigungsschritt von dem, in einer ersten aggregierten Form I vorliegenden GP 160 die an der Oberfläche befindlichen Verunreinigungen entfernt werden, GP 160 sodann unter Verwendung eines nicht-denaturierenden Detergenz in eine zweite aggregierte Form II überführt wird und in einem zweiten Reinigungsschritt die in dieser aggregierten Form II zugänglichen Verunreinigungen entfernt werden und gegebenenfalls durch Zugabe eines zweiten, nicht-denaturierenden Detergenz GP 160 in eine monomere Form III übergeführt wird und durch Entfernen des nicht-denaturierenden Detergenz reversibel wieder in die aggregierte Form I oder II überführbar ist.

16. Verfahren nach Anspruch 15, **dadurch gekennzeichnet,** daß zu Reinigungszwecken das reversible Überführen der aggregierten Formen I, II und III mehrfach durchgeführt wird.

17. Verfahren nach Anspruch 15, **dadurch gekennzeichnet,** daß das GP 160 ein Expressionsprodukt gentechnisch manipulierter Zellen, insbesondere solcher, die Proteine überproduzieren, ist.

18. Verfahren nach mindestens einem der Ansprüche 15 oder 17, **dadurch gekennzeichnet,** daß die Zellen eukaryontische, bevorzugt tierische oder humane Zellen sind.

19. Verfahren nach Anspruch 18, **dadurch gekennzeichnet,** daß die Zellen aus primären Zellkulturen oder aus Zelllinien gewonnen werden, insbesondere Verozellen, CHO-Zellen oder primäre Hühnerembryofibroblastenzellen.

20. Verfahren nach Anspruch 15, **dadurch gekennzeichnet,** daß die Detergenzien bestimmte ionische, nicht-ionische oder zwitterionische Detergenzien sind.

21. Verfahren nach Anspruch 20, **dadurch gekennzeichnet,** daß als erstes, nicht-ionisches Detergenz Octylglucosid oder ein Derivat von diesem, vorzugsweise in einer Konzentration von 0.25 bis 5%, insbesondere 1%, und als zweites, zwitterionisches, Detergenz eins der Zwittergent-Reihe, vorzugsweise in einer Konzentration von 0,25 bis 5%, insbesondere 1%, verwendet wird.

22. Verfahren nach Anspruch 20, **dadurch gekennzeichnet,** daß das Detergenz ein Salz der Gallensäure oder ein Derivat von diesem ist, bevorzugt Deoxycholat (DOC), vorzugsweise in einer Konzentration von 0,25 bis 5%, insbesondere 1%.

## Claims (Claims for the following Contracting State(s): AT, BE, CH, DE, FR, GB, IT, LI, LU, NL, SE)

1. GP 160 in loosened electronoptically visible, aggregated, electrophoretically non-mobile form (form II), obtainable by treating approximately spheroidally aggregated, compact, electronoptically visible, electrophoretically non-mobile GP 160 (form I), which is produced in cells, with a non-denaturing detergent.

2. GP 160 according to Claim 1, characterised in that it is present in at least 80%, preferably 98%, purity.

3. GP 160 according to one of Claims 1 or 2, characterised in that it is an expression product of genetically manipulated cells, in particular of those which overproduce the proteins.

4. GP 160 according to Claim 3, characterised in that it possesses an antigenic function.

5. GP 160 according to at least one of the preceding claims, characterised in that the cells are eucaryotic, preferably animal or human, cells.

6. GP 160 according to Claim 5, characterised in that the cells are obtained from primary cell cultures or from cell lines, in particular Vero cells, CHO cells or primary chick embryo fibroblast cells.

7. GP 160 according to at least one of the preceding claims, characterised in that the detergents are particular ionic, non-ionic or zwitterionic detergents.

8. GP 160 according to Claim 7, characterised in that octyl glucoside, or a derivative thereof, is used, preferably at a concentration of 0.25 to 5%, in particular 1%, as the first, non-ionic detergent for converting form I into form II, and that one of the Zwittergent series is used, preferably at a concentration of 0.25 to 5%, in particular 1%, as the second, zwitterionic detergent for converting form II into the monomeric form (form III).

9. GP 160 according to Claim 7, characterised in that the detergent is a salt of bile acid, or a derivative thereof, preferably deoxycholate (DOC), preferably at a concentration of 0.25 to 5%, in particular 1%.

10. Use of GP 160 according to one of the preceding claims in its aggregated form I for stable storage.

11. Use of GP 160 according to one of the preceding claims in its aggregated form II for reinforcing the biological function.

12. GP 160 in form II for therapeutic, prophylactic or diagnostic application.

13. Use of GP 160 in form II for obtaining vaccines.

14. Use of GP 160 in form II for obtaining substances for the qualitative detection or quantitative determination of the said substances in body fluids.

15. Process for isolating and purifying GP 160 produced in cells, characterised in that, in a first purification step, the impurities located on the surface are removed from the GP 160 present in a first aggregated form I, GP 160 is then converted into a second aggregated form II using a non-denaturing detergent and, in a second purification step, the impurities which are accessible in this aggregated form II are removed and, where appropriate, GP 160 is converted, by the addition of a second, non-denaturing detergent, into a monomeric form III and is reversibly convertible, by removing the non-denaturing detergent, once again into the aggregated form I or II.

16. Process according to Claim 15, characterised in that, for purification purposes, the reversible conversion of the aggregated forms I, II and III is carried out repeatedly.

17. Process according to Claim 15, characterised in that the GP 160 is an expression product of genetically manipulated cells, in particular of those which overproduce proteins.

18. Process according to at least one of Claims 15 or 17, characterised in that the cells are eucaryotic, preferably animal or human, cells.

19. Process according to Claim 18, characterised in that the cells are obtained from primary cell cultures or from cell lines, in particular Vero cells, CHO cells or primary chick embryo fibroblast cells.

20. Process according to Claim 15, characterised in that the detergents are particular ionic, non-ionic or zwitterionic detergents.

21. Process according to Claim 20, characterised in that octyl glucoside, or a derivative thereof, is used, preferably at a concentration of 0.25 to 5%, in particular 1%, as the first, non-ionic detergent, and that one of the Zwittergent series is used, preferably at a concentration of 0.25 to 5%, in particular 1%, as the second, zwitterionic detergent.

22. Process according to Claim 20, characterised in that the detergent is a salt of bile acid, or a derivative thereof, preferably deoxycholate (DOC), preferably at a concentration of 0.25 to 5%, in particular 1%.

## Claims (Claims for the following Contracting State(s): ES)

1. Process for preparing GP 160 in loosened electronoptically visible, aggregated, electrophoretically non-mobile form (form II), in which approximately spheroidally aggregated, compact, electronoptically visible, electrophoretically non-mobile GP 160 (form I), which is produced in cells, is treated with a non-denaturing detergent.

2. Process according to Claim 1, characterised in that it leads to a GP 160 having at least 80%, preferably 98%, purity.

3. Process according to one of Claims 1 or 2, characterised in that the GP160 is an expression product of genetically manipulated cells, in particular of those which overproduce the proteins.

4. Process according to Claim 3, characterised in that the GP 160 possesses an antigenic function.

5. Process according to at least one of the preceding claims, characterised in that the cells are eucaryotic, preferably animal or human, cells.

6. Process according to Claim 5, characterised in that the cells are obtained from primary cell cultures or from cell lines, in particular Vero cells, CHO cells or primary chick embryo fibroblast cells.

7. Process according to at least one of the preceding claims, characterised in that the detergents are particular ionic, non-ionic or zwitterionic detergents.

8. Process according to Claim 7, characterised in that octyl glucoside, or a derivative thereof, is used, preferably at a concentration of 0.25 to 5%, in particular 1%, as the first, non-ionic detergent for converting form I into form II, and that one of the Zwittergent series is used, preferably at a concentration of 0.25 to 5%, in particular 1%, as the second, zwitterionic detergent for converting form II into the monomeric form (form III).

9. Process according to Claim 7, characterised in that the detergent is a salt of bile acid, or a derivative thereof, preferably deoxycholate (DOC), preferably at a concentration of 0.25 to 5%, in particular 1%.

10. Use of GP 160 according to one of the preceding claims in its aggregated form I for stable storage.

11. Use of GP 160 according to one of the preceding claims in its aggregated form II for reinforcing the biological function.

12. Process for preparing a composition for therapeutic, prophylactic or diagnostic application, characterised in that GP 160 in form II is combined, in a manner which is customary per se, with additives, vehicles or diluents.

13. Use of GP 160 in form II for obtaining vaccines.

14. Use of GP 160 in form II for obtaining substances for the qualitative detection or quantitative determination of the said substances in body fluids.

15. Process for isolating and purifying GP 160 produced in cells, characterised in that, in a first purification step, the impurities located on the surface are removed from the GP 160 present in a first aggregated form I, GP 160 is then converted into a second aggregated form II using a non-denaturing detergent and, in a second purification step, the impurities which are accessible in this aggregated form II are removed and, where appropriate, GP 160 is converted, by the addition of a second, non-denaturing detergent, into a monomeric form III and is reversibly convertible, by removing the non-denaturing detergent, once again into the aggregated form I or II.

16. Process according to Claim 15, characterised in that, for purification purposes, the reversible conversion of the aggregated forms I, II and III is carried out repeatedly.

17. Process according to Claim 15, characterised in that the GP 160 is an expression product of genetically manipulated cells, in particular of those which over-produce proteins.

18. Process according to at least one of Claims 15 or 17, characterised in that the cells are eucaryotic, preferably animal or human, cells.

19. Process according to Claim 18, characterised in that the cells are obtained from primary cell cultures or from cell lines, in particular Vero cells, CHO cells or primary chick embryo fibroblast cells.

20. Process according to Claim 15, characterised in that the detergents are particular ionic, non-ionic or zwitterionic detergents.

21. Process according to Claim 20, characterised in that octyl glucoside, or a derivative thereof, is used, preferably at a concentration of 0.25 to 5%, in particular 1%, as the first, non-ionic detergent, and that one of the Zwittergent series is used, preferably at a concentration of 0.25 to 5%, in particular 1%, as the second, zwitterionic detergent.

22. Process according to Claim 20, characterised in that the detergent is a salt of bile acid, or a derivative thereof, preferably deoxycholate (DOC), preferably at a concentration of 0.25 to 5%, in particular 1%.

## Claims (Claims for the following Contracting State(s): GR)

1. GP 160 in loosened electronoptically visible, aggregated, electrophoretically non-mobile form (form II), obtainable by treating approximately spheroidally aggregated, compact, electronoptically visible, electrophoretically non-mobile GP 160 (form I), which is produced in cells, with a non-denaturing detergent.

2. GP 160 according to Claim 1, characterised in that it is present in at least 80%, preferably 98%, purity.

3. GP 160 according to one of Claims 1 or 2, characterised in that it is an expression product of genetically manipulated cells, in particular of those which overproduce the proteins.

4. GP 160 according to Claim 3, characterised in that it possesses an antigenic function.

5. GP 160 according to at least one of the preceding claims, characterised in that the cells are eucaryotic, preferably animal or human, cells.

6. GP 160 according to Claim 5, characterised in that the cells are obtained from primary cell cultures or from cell lines, in particular Vero cells, CHO cells or primary chick embryo fibroblast cells.

7. GP 160 according to at least one of the preceding claims, characterised in that the detergents are particular ionic, non-ionic or zwitterionic detergents.

8. GP 160 according to Claim 7, characterised in that octyl glucoside, or a derivative thereof, is used, preferably at a concentration of 0.25 to 5%, in particular 1%, as the first, non-ionic detergent for converting form I into form II, and that one of the Zwittergent series is used, preferably at a concentration of 0.25 to 5%, in particular 1%, as the second, zwitterionic detergent for converting form II into the monomeric form (form III).

9. GP 160 according to Claim 7, characterised in that the detergent is a salt of bile acid, or a derivative thereof, preferably deoxycholate (DOC), preferably at a concentration of 0.25 to 5%, in particular 1%.

10. Use of GP 160 according to one of the preceding claims in its aggregated form I for stable storage.

11. Use of GP 160 according to one of the preceding claims in its aggregated form II for reinforcing the biological function.

12. Process for preparing a composition for therapeutic, prophylactic or diagnostic application, characterised in that GP 160 in form II is combined, in a manner which is customary per se, with additives, vehicles or diluents.

13. Use of GP 160 in form II for obtaining vaccines.

14. Use of GP 160 in form II for obtaining substances for the qualitative detection or quantitative determination of the said substances in body fluids.

15. Process for isolating and purifying GP 160 produced in cells, characterised in that, in a first purification step, the impurities located on the surface are removed from the GP 160 present in a first aggregated form I, GP 160 is then converted into a second aggregated form II using a non-denaturing detergent and, in a second purification step, the impurities which are accessible in this aggregated form II are removed and, where appropriate, GP 160 is converted, by the addition of a second, non-denaturing detergent, into a monomeric form III and is reversibly convertible, by removing the non-denaturing detergent, once again into the aggregated form I or II.

16. Process according to Claim 15, characterised in that, for purification purposes, the reversible conversion of the aggregated forms I, II and III is carried out repeatedly.

17. Process according to Claim 15, characterised in that the GP 160 is an expression product of genetically manipulated cells, in particular of those which over-produce proteins.

18. Process according to at least one of Claims 15 or 17, characterised in that the cells are eucaryotic, preferably animal or human, cells.

19. Process according to Claim 18, characterised in that the cells are obtained from primary cell cultures or from cell lines, in particular Vero cells, CHO cells or primary chick embryo fibroblast cells.

20. Process according to Claim 15, characterised in that the detergents are particular ionic, non-ionic or zwitterionic detergents.

21. Process according to Claim 20, characterised in that octyl glucoside, or a derivative thereof, is used, preferably at a concentration of 0.25 to 5%, in particular 1%, as the first, non-ionic detergent, and that one of the Zwittergent series is used, preferably at a concentration of 0.25 to 5%, in particular 1%, as the second, zwitterionic detergent.

22. Process according to Claim 20, characterised in that the detergent is a salt of bile acid, or a derivative thereof, preferably deoxycholate (DOC), preferably at a concentration of 0.25 to 5%, in particular 1%.

## Revendications (Revendications pour l'(les) Etat(s) contractant(s) suivant(s): AT, BE, CH, DE, FR, GB, IT, LI, LU, NL, SE)

1. GP 160 sous une forme lâche, visible électronooptiquement, agrégée, électrophorétiquement non-mobile (forme II), pouvant être obtenu par traitement d'un GP 160 produit dans des cellules, agrégé d'une manière légèrement sphéroïdale, compact, visible électronooptiquement, électrophorétiquement non-mobile (forme I) avec un détergent non-dénaturant.

2. GP 160 selon la revendication 1, caractérisé en ce qu'il est présent à une pureté d'au moins 80 %, de préférence de 98 %.

3. GP 160 selon l'une des revendications 1 ou 2, caractérisé en ce qu'il est un produit d'expression de cellules manipulées par la technique génétique, en particulier de cellules surproduisant les protéines.

4. GP 160 selon la revendication 3, caractérisé en ce qu'il présente une fonction antigénique.

5. GP 160 selon au moins l'une des revendications précédentes, caractérisé en ce que les cellules sont des cellules eucaryotes, de préférence des cellules animales ou humaines.

6. GP 160 selon la revendication 5, caractérisé en ce que les cellules sont obtenues à partir de cultures cellulaires primaires ou à partir de lignées cellulaires, en particulier de cellules Vero, de cellules CHO ou de fibroblastes d'embryons de poulet primaires.

7. GP 160 selon au moins l'une des revendications précédentes, caractérisé en ce que les détergents sont des détergents ioniques, non-ioniques ou amphotères déterminés.

8. GP 160 selon la revendication 7, caractérisé en ce qu'on utilise comme premier détergent non-ionique pour la transformation de la forme I en la forme II un octylglucoside ou un dérivé de celui-ci, de préférence à une concentration de 0,25 à 5 %, en particulier de 1 %, et comme second détergent amphotère pour la transformation de la forme II en la forme monomère (forme III) un détergent de la série des détergents Zwittergent, de préférence à une concentration de 0,25 à 5 %, en particulier de 1 %.

9. GP 160 selon la revendication 7, caractérisé en ce que le détergent est un sel de l'acide gallique ou un dérivé de celui-ci, de préférence le désoxycholate (DOC), de préférence à une concentration de 0,25 à 5 %, en particulier de 1 %.

10. Utilisation du GP 160 selon l'une des revendications précédentes sous sa forme agrégée I pour un stockage stable.

11. Utilisation du GP 160 selon l'une des revendications précédentes sous sa forme agrégée II pour le renforcement de la fonction biologique.

12. GP 160, sous la forme II pour l'utilisation thérapeutique, prophylactique ou diagnostique.

13. Utilisation du GP 160 sous la forme II pour la préparation du vaccin.

14. Utilisation du GP 160 sous la forme II pour la préparation de substances pour la détection qualitative ou la détermination quantitative des substances indiquées dans des fluides corporels.

15. Procédé pour l'isolement et la purification d'un GP 160 produit dans des cellules, caractérisé en ce que dans une première étape de purification du GP 160 présent sous une première forme agrégée I, on élimine les impuretés présentes à la surface, puis on transforme le GP 160 en utilisant un détergent non dénaturant en une seconde forme agrégée II, et, dans une deuxième étape de purification, on élimine les impuretés accessibles dans cette forme agrégée II et on transforme le cas échéant, par addition d'un deuxième détergent non dénaturant, le GP 160 en une forme monomère III, et par élimination du détergent non dénaturant, on peut le retransformer dans la forme agrégée I ou II.

16. Procédé selon la revendication 15, caractérisé en ce qu'à des fins de purification, on effectue plusieurs fois la transformation réversible des formes agrégées I, II et III.

17. Procédé selon la revendication 15, caractérisé en ce que le GP 160 est un produit d'expression de cellules manipulées par la technique génétique, en particulier de cellules qui surproduisent des protéines.

18. Procédé selon au moins l'une des revendications 15 ou 17, caractérisé en ce que les cellules sont des cellules eucaryotes, de préférence des cellules animales ou humaines.

19. Procédé selon la revendication 18, caractérisé en ce que les cellules sont obtenues à partir de cultures cellulaires primaires ou de lignées cellulaires, en particulier de cellules Vero, de cellules CHO ou de fibroblastes d'embryons de poulet primaires.

20. Procédé selon la revendication 15, caractérisé en ce que les détergents sont des détergents ioniques, non-ioniques ou amphotères déterminés.

21. Procédé selon la revendication 20, caractérisé en ce qu'on utilise comme premier détergent non-ionique un octylglucoside ou un dérivé de celui-ci, de préférence à une concentration de 0,25 à 5 %, en particulier de 1 %, et comme second détergent amphotère, un détergent de la série des détergents Zwittergent, de préférence à une concentration de 0,25 à 5 %, en particulier de 1 %.

22. Procédé selon la revendication 20, caractérisé en ce que le détergent est un sel de l'acide biliaire ou un dérivé de celui-ci, de préférence le désoxycholate (DOC), de préférence à une concentration de 0,25 à 5 %, en particulier de 1 %.

## Revendications (Revendications pour l'(les) Etat(s) contractant(s) suivant(s): ES)

1. Procédé de préparation du GP 160 sous une forme lâche, visible électrono-optiquement, agrégée, électrophorétiquement non-mobile (forme II), dans lequel le GP 160 produit dans les cellules, agrégé d'une manière légèrement sphéroïdale, compacte, visible électronooptiquement, électrophorétiquement non-mobile (forme I) est traité par un détergent non-dénaturant.

2. Procédé selon la revendication 1, caractérisé en ce qu'il conduit à un GP 160 ayant une pureté d'au moins 80 %, de préférence de 98 %.

3. Procédé selon l'une des revendications 1 ou 2, caractérisé en ce que le GP 160 est un produit d'expression de cellules manipulées par la technique génétique, en particulier de cellules qui surproduisent les protéines.

4. Procédé selon la revendication 3, caractérisé en ce que le GP 160 présente une fonction antigénique.

5. Procédé selon au moins l'une des revendications précédentes, caractérisé en ce que les cellules sont des cellules eucaryotes, de préférence des cellules animales ou humaines.

6. Procédé selon la revendication 5, caractérisé en ce que les cellules ont été obtenues à partir de cultures cellulaires primaires ou à partir de lignées cellulaires, en particulier de cellules Vero, de cellules CHO ou de fibroblastes d'embryons de poulet.

7. Procédé selon au moins l'une des revendications précédentes, caractérisé en ce que les détergents sont des détergents ioniques, non-ioniques ou amphotères déterminés.

8. Procédé selon la revendication 7, caractérisé en ce qu'on utilise comme premier détergent non-ionique pour la transformation de la forme I en la forme II un octylglucoside ou un dérivé de celui-ci, de préférence à une concentration de 0,25 à 5 %, en particulier de 1 %, et comme second détergent amphotère pour la transformation de la forme II en la forme monomère (forme III) un détergent de la série des détergents Zwittergent, de préférence à une concentration de 0,25 à 5 %, en particulier de 1 %.

9. Procédé selon la revendication 7, caractérisé en ce que le détergent est un sel de l'acide biliaire ou un dérivé de celui-ci, de préférence le désoxycholate (DOC), de préférence à une concentration de 0,25 à 5 %, en particulier de 1 %.

10. Utilisation du GP 160 selon l'une des revendications précédentes sous sa forme agrégée I pour un stockage stable.

11. Utilisation du GP 160 selon l'une des revendications précédentes sous sa forme agrégée II pour le renforcement de la fonction biologique.

12. Procédé de préparation d'une composition pour l'utilisation thérapeutique, prophylactique ou diagnostique, caractérisé en ce que le GP 160, sous la forme II, est combiné d'une manière habituelle en soi avec des additifs, des supports ou des diluants.

13. Utilisation du GP 160 sous la forme II pour la préparation du vaccin.

14. Utilisation du GP 160 sous la forme II pour la préparation de substances pour la détection qualitative ou la détermination quantitative des substances indiquées dans des fluides corporels.

15. Procédé pour l'isolement et la purification d'un GP 160 produit dans des cellules, caractérisé en ce que dans une première étape de purification du GP 160 présent sous une première forme agrégée I, on élimine les impuretés présentes à la surface, puis on transforme le GP 160 en utilisant un détergent non dénaturant en une seconde forme agrégée II, et, dans une deuxième étape de purification, on élimine les impuretés accessibles dans cette forme agrégée II et on transforme le cas échéant, par addition d'un deuxième détergent non dénaturant, le GP 160 en une forme monomère III, et par élimination du détergent non dénaturant, on peut le retransformer dans la forme agrégée I ou II.

16. Procédé selon la revendication 15, caractérisé en ce qu'à des fins de purification, on effectue plusieurs fois la transformation réversible des formes agrégées I, II et III.

17. Procédé selon la revendication 15, caractérisé en ce que le GP 160 est un produit d'expression de cellules manipulées par la technique génétique, en particulier de cellules qui surproduisent des protéines.

18. Procédé selon au moins l'une des revendications 15 ou 17, caractérisé en ce que les cellules sont des cellules eucaryotes, de préférence des cellules animales ou humaines.

19. Procédé selon la revendication 18, caractérisé en ce que les cellules ont été obtenues à partir de cultures cellulaires primaires ou de lignées cellulaires, en particulier de cellules Vero, de cellules CHO ou de fibroblastes d'embryons de poulet primaires.

20. Procédé selon la revendication 15, caractérisé en ce que les détergents sont des détergents ioniques, non-ioniques ou amphotères déterminés.

21. Procédé selon la revendication 20, caractérisé en ce qu'on utilise comme premier détergent non-ionique un octylglucoside ou un dérivé de celui-ci, de préférence à une concentration de 0,25 à 5 %, en particulier de 1 %, et comme second détergent amphotère, un détergent de la série des détergents Zwittergent, de préférence à une concentration de 0,25 à 5 %, en particulier de 1 %.

22. Procédé selon la revendication 20, caractérisé en ce que le détergent est un sel de l'acide biliaire ou un dérivé de celui-ci, de préférence le désoxycholate (DOC), de préférence à une concentration de 0,25 à 5 %, en particulier de 1 %.

## Revendications (Revendications pour l'(les) Etat(s) contractant(s) suivant(s): GR)

1. GP 160 sous une forme lâche, visible électronooptiquement, agrégée, électrophorétiquement non-mobile (forme II), pouvant être obtenu par traitement d'un GP 160 produit dans des cellules, agrégé d'une manière légèrement sphéroïdale, compacte, visible électronooptiquement, électrophorétiquement non-mobile (forme I) par un détergent non-dénaturant.

2. GP 160 selon la revendication 1, caractérisé en ce qu'il est présent à une pureté d'au moins 80 %, de préférence de 98 %.

3. GP 160 selon l'une des revendications 1 ou 2, caractérisé en ce qu'il est un produit d'expression de cellules manipulées par la technique génétique, en particulier de cellules surproduisant les protéines.

4. GP 160 selon la revendication 3, caractérisé en ce qu'il présente une fonction antigénique.

5. GP 160 selon au moins l'une des revendications précédentes, caractérisé en ce que les cellules sont des cellules eucaryotes, de préférence des cellules animales ou humaines.

6. GP 160 selon la revendication 5, caractérisé en ce que les cellules sont obtenues à partir de cultures cellulaires primaires ou à partir de lignées cellulaires, en particulier de cellules Vero, de cellules CHO ou de fibroblastes d'embryons de poulet primaires.

7. GP 160 selon au moins l'une des revendications précédentes, caractérisé en ce que les détergents sont des détergents ioniques, non-ioniques ou amphotères déterminés.

8. GP 160 selon la revendication 7, caractérisé en ce qu'on utilise comme premier détergent non-ionique pour la transformation de la forme I en la forme II un octylglucoside ou un dérivé de celui-ci, de préférence à une concentration de 0,25 à 5 %, en particulier de 1 %, et comme second détergent amphotère pour la transformation de la forme II en la forme monomère (forme III) un détergent de la série des détergents Zwittergent, de préférence à une concentration de 0,25 à 5 %, en particulier de 1 %.

9. GP 160 selon la revendication 7, caractérisé en ce que le détergent est un sel de l'acide biliaire ou un dérivé de celui-ci, de préférence le désoxycholate (DOC), de préférence à une concentration de 0,25 à 5 %, en particulier de 1 %.

10. Utilisation du GP 160 selon l'une des revendications précédentes sous sa forme agrégée I pour un stockage stable.

11. Utilisation du GP 160 selon l'une des revendications précédentes sous sa forme agrégée II pour le renforcement de la fonction biologique.

12. Procédé de préparation d'une composition pour l'utilisation thérapeutique, prophylactique ou diagnostique, caractérisé en ce qu'on associe le GP 160 sous la forme II, d'une manière connue en soi, à des additifs, des supports ou des diluants.

13. Utilisation du GP 160 sous la forme II pour la préparation du vaccin.

14. Utilisation du GP 160 sous la forme II pour la préparation de substances pour la détection qualitative ou la détermination quantitative des substances indiquées dans des fluides corporels.

15. Procédé pour l'isolement et la purification d'un GP 160 produit dans des cellules, caractérisé en ce que dans une première étape de purification du GP 160 présent sous une première forme agrégée I, on élimine les impuretés présentes à la surface, puis on transforme le GP 160 en utilisant un détergent non dénaturant sous une seconde forme agrégée II, et, dans une deuxième étape de purification, on élimine les impuretés accessibles dans cette forme agrégée II et on transforme le cas échéant, par addition d'un deuxième détergent non dénaturant, le GP 160 en une forme monomère III, et par élimination du détergent non dénaturant, on peut le retransformer dans la forme agrégée I ou II.

16. Procédé selon la revendication 15, caractérisé en ce qu'à des fins de purification, on effectue plusieurs fois la transformation réversible des formes agrégées I, II et III.

17. Procédé selon la revendication 15, caractérisé en ce que le GP 160 est un produit d'expression de cellules manipulées par la technique génétique, en particulier de cellules qui surproduisent des protéines.

18. Procédé selon au moins l'une des revendications 15 ou 17, caractérisé en ce que les cellules sont des cellules eucaryotes, de préférence des cellules animales ou humaines.

19. Procédé selon la revendication 18, caractérisé en ce que les cellules sont obtenues à partir de cultures cellulaires primaires ou de lignées cellulaires, en particulier de cellules Vero, de cellules CHO ou de fibroblastes d'embryons de poulet primaires.

20. Procédé selon la revendication 15, caractérisé en ce que les détergents sont des détergents ioniques, non-ioniques ou amphotères déterminés.

21. Procédé selon la revendication 20, caractérisé en ce qu'on utilise comme premier détergent non-ionique un octylglucoside ou un dérivé de celui-ci, de préférence à une concentration de 0,25 à 5 %, en particulier de 1 %, et comme second détergent amphotère, un détergent de la série des détergents Zwittergent, de préférence à une concentration de 0,25 à 5 %, en particulier de 1 %.

22. Procédé selon la revendication 20, caractérisé en ce que le détergent est un sel de l'acide biliaire ou un dérivé de celui-ci, de préférence le désoxycholate (DOC), de préférence à une concentration de 0,25 à 5 %, en particulier de 1 %.
